# EUROPEAN PATENT SPECIFICATION

(11) **EP 3 038 631 B1**
(45) Date of publication and mention of the grant of the patent: **11.07.2018**
(21) Application number: 14840203.5
(22) Date of filing: 29.08.2014
(51) Int. Cl.: A61K 35/64, A61P 1/00, A61P 17/00, A61P 29/00, A61K 35/644, C07K 14/435, G01N 33/50, G01N 33/68, A61K 9/00

(54) **ANTI-INFLAMMATORY COMPOSITIONS, METHODS AND USES THEREOF**
ENTZÜNDUNGSHEMMENDE ZUSAMMENSETZUNGEN, VERFAHREN UND VERWENDUNGEN DAVON
COMPOSITIONS ANTI-INFLAMMATOIRES, MÉTHODES ET UTILISATIONS CORRESPONDANTES

(30) Priority: 30.08.2013 NZ 61491213
(43) Date of publication of application: 06.07.2016
(73) Proprietor: Apimed Medical Honey Limited, Paengaroa 3189 (NZ)
(72) Inventor: KUHNE, Jennifer, Paengaroa 3189 (NZ); STEINHORN, Gregor Aaron, Paengaroa 3189 (NZ); TAYLOR, John Alexander, Paengaroa 3189 (NZ)
(74) Representative: CSY St Albans
(86) International application number: PCT/NZ2014/000182
(87) International publication number: WO 2015/030609

(56) References cited:
- WO-A1-2013/061816
- WO-A2-2012/087160
- AU-A1- 2013 202 889
- JP-A- 2005 112 785
- KASSIM M. ET AL.: 'Ellagic acid, phenolic acids, and flavonoids in Malaysian honey extracts demonstrate in vitro anti-inflammatory activity' NUTRITION RESEARCH vol. 30, no. 9, September 2010, pages 650 - 659, XP027406929
- KASSIM M. ET AL.: 'The inhibitory effects of Gelam honey and its extracts on nitric oxide and prostaglandin E2 in inflammatory tissues' FITOTERAPIA vol. 81, no. 8, December 2010, pages 1196 - 1201, XP027487794
- TONKS A.J. ET AL.: 'A 5.8-kDa component of manuka honey stimulates immune cells via TLR4' JOURNAL OF LEUKOCYTE BIOLOGY vol. 82, no. 5, pages 1147 - 1155, XP009101128
- ETERAF-OSKOUEI T. ET AL.: 'Traditional and Modem Uses of Natural Honey in Human Diseases: A Review' IRAN J BASIC MED SCI. vol. 16, no. 6, June 2013, pages 731 - 742, XP055320724
- LEONG AG1 ET AL: "Indigenous New Zealand honeys exhibit multiple anti-inflammatory activities", INNATE IMMU, SAGE PUBLICATIONS LTD, vol. 18, no. 3, 6 October 2011 (2011-10-06), pages 459-466, XP008175942, ISSN: 1753-4259, DOI: 10.1177/1753425911422263
- STEPHENS J M ET AL: "Phenolic compounds and methylglyoxal in some New Zealand manuka and kanuka honeys", FOOD CHEMISTRY, ELSEVIER LTD, NL, vol. 120, no. 1, 1 May 2010 (2010-05-01), pages 78-86, XP026799324, ISSN: 0308-8146 [retrieved on 2009-09-26]
- VICTORIA TOMBLIN ET AL: "Potential pathway of anti-inflammatory effect by New Zealand honeys", INTERNATIONAL JOURNAL OF GENERAL MEDICINE, 5 March 2014 (2014-03-05), pages 149-158, XP055421892, DOI: 10.2147/IJGM.S45839

## Description

### TECHNICAL FIELD

Described herein are anti-inflammatory compositions, methods and uses thereof. More specifically, anti-inflammatory compounds, medicaments containing fractions and/or compounds, methods of fractionation, methods of use and methods of testing are described herein.

### BACKGROUND ART

Immunostimulatory compounds are compounds that can encourage cytokine production and hence macrophage production, all being part of a normal immune system reaction observed in organisms. The main effects of immunostimulatory compounds result in the migration of macrophages to an inflamed area and an increase in (already existing) macrophage activity.

Anti-inflammatory compounds are compounds that have the opposite effect to the above effectively stopping or slowing the stimulation going too far and preventing the potential of a cascade in stimulation, in worst cases leading to systemic inflammatory response syndrome (SIRS), also related to sepsis.

Anti-inflammatories are well known and used in medicine, the aim being to calm the immune system. Inflammation relating to immune stimulation is often considered a negative reaction or a reaction to be avoided; particularly in the context of wound healing hence administration of an anti-inflammatory compound or compounds is a common form of treatment.

Honey is well known to have anti-microbial effects both due to the peroxide levels and so-called non-peroxide effects. By way of example regarding non-peroxide effects, some honeys such as those from the genus *Leptospermum spp.* have so called non-perodixe effects in part or in whole attributable to the presence of the compound methylglyoxal (MGO). Beyond the anti-microbial effects of honey there are also many other compounds in honey that have immune system modulating effects.

Honey derived arabinogallactan protein (AGP) compounds have been described in earlier patent applications by the applicant including WO2011/139168A1 and WO2013/157961A1. These compounds have been identified in the applicant's research as having immune stimulation effects and as such may be useful in the production of medicaments, food supplements or cosmetics to stimulate the immune system. One practical use for these compounds may be to stimulate the immune system of a patient in a chronic slow healing wound so as to kick start the immune system into healing properly again.

Apisimin is one of three key functional proteins naturally found in royal jelly and is another compound identified by the applicant as having immune stimulation effects as noted in WO2013/157961A1.

Anti-inflammatory effects from honey have been suspected - this may be one reason for their use in medical dressings. The anti-inflammatory effects are somewhat forgotten in the art, the art focussing on the anti-microbial effects of honey - effects that are important, but this narrow focus does not always tell the full story of why honey is useful as a medicament. Eteraf-Oskouei et al. 2013 "Traditional and modern uses of natural honey in human diseases: a review", Iran J Basic Med Sci, vol. 16, no. 6, June 2013, pages 731-742, discusses traditional and modern uses of natural honey in human diseases, such as an anti-inflammatory agent.

Leong et al, Innate Immunity, vol. 18, no. 3, 6 October 2011, pages 459-466, discusses anti-inflammatory activity in indigenous New Zealand honeys.

WO2012/087160 relates to anti-inflammatory proteins, their uses, methods of preparation and methods of their detection. In particular, it relates to major royal jelly protein MRJP1 modified by MGO and fragments thereof from manuka honey.

One patent publication by the applicant published as WO2010/082846A1 first considered that anti-inflammatory effects might be present in honey. The publication teaches about concentrating the level of phenolic compounds in honey (particularly methoxylated phenolic compounds) in order to emphasise the honey anti-inflammatory activity. Other methods of enhancing the anti-inflammatory effects of honey described include aging the honey and the addition of tannase enzyme to the honey. A specific fraction of compounds or compounds other than phenolics in honey, were not identified. Stephens et al, Food Chemistry, vol 120, no. 1, 1 May 2010, pages 78-86 and Tomblin et al, International Journal of General Medicine, 5 March 2014, pages 149-158, both discuss phenolic compounds and anti-inflammatory activity.

Some art purports to have shown that selected glycoside compounds have anti-inflammatory effects. WO2003/047599 describes the glycoside catalepsoide as an anti-inflammatory and WO2013/061816 describes a honey derived compound termed leptosin as an anti-inflammatory compound. These publications describe the actions of a specific component for a specific biological action. Specifically, WO2013/061816 teaches about an anti-inflammatory effect of leptosin due to myeloperoxidase inhibition, this being only one form of anti-inflammatory action. More generalised anti-inflammatory effects as described herein from a wider fraction of compounds cannot be directly explained by the sole action of leptosin on myeloperoxidase. Similarly, WO2003/047599 concentrates on catalposide and again, a specific form of inflammatory inhibition is contemplated and not a generalised effect from potentially a number of compounds. Similarly, Kassim et al. 2010 "Ellagic acid, phenolic acids, and flavonoids in Malaysian honey extracts demonstrate in vitro anti-inflammatory activity", Nutrition Research, vol. 30, no. 9, September 2010, pages 650-659, and Kassim et al. 2010, "The inhibitory effects of Gelam honey and its extracts on nitric oxide and prostaglandin E 2 in inflammatory tissues", Fitoterapia, vol. 81 no. 8, December 2010, pages 1196-1201, concentrate on the anti-inflammatory activity of phenolic compounds in Malaysian honey.

Two other publications, Gannabathula 2011 and Tonks et. al. 2007, discuss aspects about producing a low molecular weight fraction from honey but both do this from the perspective of showing inflammatory effects. Neither publication describes or even contemplates that a low molecular weight fraction of this nature would have anti-inflammatory effects.

It should be appreciated from the above that it would be useful to identify a specific fraction of compounds or specific compounds in a honey that may be isolated and used in various forms as an anti-inflammatory agent. Methods of testing honeys to identify highly anti-inflammatory honeys may also be of benefit or at least provide the public with a useful choice.

Further aspects and advantages of the anti-inflammatory fractions and compounds, medicaments containing the fractions and/or compounds, methods of fractionation, methods of use and methods of testing will become apparent from the ensuing description that is given by way of example only.

### SUMMARY

Described herein are anti-inflammatory fractions and compounds, medicaments containing the fractions and/or compounds, methods of fractionation, methods of use and methods of testing. The inventors have identified that a low molecular weight fraction from honey has strong and generalised anti-inflammatory effects and no immune-stimulatory effects. Being able to isolate compounds with anti-inflammatory activity allows the ability to produce medicaments for various uses including medical products, fortified foods, supplements and cosmetics. In addition, a generalised anti-inflammatory effect may be more useful than just a singular focus on one aspect of inflammation such as myeloperoxidase inhibition. The invention is defined by the claims.

Advantages of the above anti-inflammatory fraction containing methods of use, methods of isolation and methods of analysis may be varied. The source of the active compounds is a naturally occurring product able to be manufactured on a sustainable basis. The fraction, at least in a concentrated form, is not anticipated to have side effects. The fraction may be formulated in a wide variety of ways for various methods of administration. Further the anti-inflammatory effects are significant and generalised suggesting good efficacy and a wide range of use.

### BRIEF DESCRIPTION OF THE DRAWINGS

Further aspects of the fractions, compositions, methods and uses will become apparent from the following description that is given by way of example only and with reference to the accompanying drawings in which:
- Figure 1: illustrates a graph showing the cytotoxic effects crude honeys at varying dilutions have on the tested RAW 264.7 cell culture and how this effect is independent of sugar content;
- Figure 2: illustrates a graph showing the cytotoxicity effects of high and low molecular weight fractions from honey on the tested RAW 264.7 cell culture;
- Figure 3: illustrates a graph showing the immune stimulation effects of a crude honey on the tested cell culture as measured via nitric oxide production by the RAW 264.7 cells;
- Figure 4: illustrates a graph showing the immune stimulation effects of a crude honey on the tested cell culture as measured via TNF-α production by the RAW 264.7 cells;
- Figure 5: illustrates a graph showing the immune stimulation effects of a >30kDa fraction from honey on the tested cell culture as measured via nitric oxide production by the RAW 264.7 cells;
- Figure 6: illustrates a graph showing the immune stimulation effects of a >30kDa fraction from honey on the tested cell culture as measured via TNF-α production by the RAW 264.7 cells;
- Figure 7: illustrates a graph showing the immune stimulation effects of a <10kDa fraction from honey on the tested cell culture as measured via nitric oxide production by the RAW 264.7 cells;
- Figure 8: illustrates a graph showing the immune stimulation effects of a <10kDa fraction from honey on the tested cell culture as measured via TNF-α production by the RAW 264.7 cells;
- Figure 9: illustrates a graph showing the anti-inflammatory effects of a <10kDa fraction from honey as measured via the reduction in LPS induced production of nitric oxide from RAW 264.7 cells;
- Figure 10: illustrates a graph showing the anti-inflammatory effects of a <10kDa fraction from honey as measured via the reduction in LPS induced production of IL-6 production from RAW 264.7 cells;
- Figure 11: illustrates a graph showing the oxidative burst reaction in vitro as measured via superoxide production based on increased whole honey concentration;
- Figure 12: illustrates a graph showing the oxidative burst reaction in vitro as measured via superoxide production based on increased >30kDa honey fraction concentration;
- Figure 13: illustrates a graph showing the oxidative burst reaction in vitro as measured via superoxide production based on increased <10kDa honey fraction concentration;
- Figure 14: illustrates a graph showing the oxidative burst reaction in vitro as measured via superoxide production based on a purified <10kDa honey fraction concentration;
- Figure 15: illustrates a graph showing the oxidative burst reaction in vitro as measured via superoxide production based on a purified <10kDa honey fraction concentration;
- Figure 16: illustrates a graph showing the nitric oxide inhibition effects from a <10kDa fraction; and
- Figure 17: illustrates a graph showing the nitric oxide inhibition effects from a purified <10kDa fraction.

### DETAILED DESCRIPTION

As noted above, described herein are anti-inflammatory fractions and compounds, medicaments containing the fractions and/or compounds, methods of fractionation, methods of use and methods of testing.

For the purposes of this specification, the term 'about' or 'approximately' and grammatical variations thereof mean a quantity, level, degree, value, number, frequency, percentage, dimension, size, amount, weight or length that varies by as much as 30, 25, 20, 15, 10, 9, 8, 7, 6, 5, 4, 3, 2, or 1% to a reference quantity, level, degree, value, number, frequency, percentage, dimension, size, amount, weight or length.

The term 'comprise' and grammatical variations thereof shall have an inclusive meaning - i.e. that it will be taken to mean an inclusion of not only the listed components it directly references, but also other non-specified components or elements.

The term 'substantially' refers to at least about 50%, for example 75%, 85%, 95% or 98%.

The term 'honey analogue' refers to a mixture of 30-50% glucose, 30-50% fructose, 1-18% water and either or both of glucose oxidase enzyme and/or hydrogen peroxide. Where the analogue is used shortly after production, hydrogen peroxide itself may be used. Where the analogue may be stored for a period of time, the analogue by preference contains glucose oxidase enzyme. As may be appreciated, glucose oxidase enzyme converts sugars into hydrogen peroxide that also results in a lower pH. If hydrogen peroxide alone is used and then the analogue stored, it is possible that the peroxide level will decrease by normal reduction equilibrium and the pH level then increase. Using glucose oxidase enzyme ensures a steady level of hydrogen peroxide and hence steady pH. The quantities used are intended to approximate the composition of naturally produced honey.

The term 'honey fraction' refers to portion of a naturally produced honey.

The term 'gelling agent' or grammatical variations thereof refers to an agent that, in the absence of liquid is not a gel, but the agent is able to form a gel in the presence of liquid.

The term 'dressing' refers to any covering that may be applied to a lesion where lesions encompass infected and non-infected abrasions, cuts, bits, burns, wounds, ulcers, abscesses, surgical wounds, fungating tumours and pressure sores.

The term 'therapeutically effective' with reference to an amount or dosage of a composition or medicament noted refers to an amount of a composition that is sufficient to effectively suppress in part or in whole, stimulation the immune system of a subject. However, this term should not be seen as limiting as 'therapeutically effective' may refer to an amount or dosage of a composition or medicament that optimises the anti-inflammatory effects on a subject depending on desired application.

The term 'isolate' or grammatical variations thereof refers to a composition containing compounds separated or isolated from a honey.

The term 'anti-inflammatory' or grammatical variations thereof refer to the subject's immune system being quenched, calmed or suppressed to the extent that macrophage cells are either no longer present at a wound site or equivalent and/or where macrophage cells if present no longer produce or at least produced a reduced amount of cytokines consistent with an inflammatory response including but not limited to TNF-α, IL-6 and IL-10 - the reduced amount being relative to the situation where no anti-inflammatory compound or compounds were added.

The term 'generalised' or grammatical variations thereof, in the context of inflammation described herein, refers to effects that influence several or a majority of all inflammatory reactions of an immune cell or organism. This is in contrast to specific anti-inflammatory effects in which one specific inflammatory response is inhibited. A generalised anti-inflammatory effect described herein will inhibit pathogen detection or inflammatory intra-cell and/or inter-cell signalling in such a way that several or all inflammatory responses are prevented or reduced.

The term 'topical' refers to placement on a body area of a subject such as skin as well as mucosal areas such as the oral cavity e.g. gums, the nasal cavity and the vaginal cavity. The term may also encompass the intestine wall.

The term 'sensitive' or grammatical variations thereof refer to a skin area that the subject finds particularly painful.

The term 'medicament' or grammatical variations thereof refers to medical products such as wound dressings, medicinal creams, gels or ointments. The term also encompasses fortified foods or supplements and cosmetic products. Disclosed herein is a method of treating a skin condition on a subject in need thereof by the step of administering a medicament to an affected skin site on a subject in need thereof, the medicament having anti-inflammatory activity and comprising as an active, a less than or equal to 10kDa fraction obtained from honey. Also disclosed herein is a method of treating a stomach ulcer and/or a digestive condition in a subject in need thereof by the step of administering a medicament to the subject, the medicament having anti-inflammatory activity and comprising as an active, a less than or equal to 10kDa fraction obtained from honey. Also disclosed herein is the use of an anti-inflammatory activity less than or equal to 10kDa fraction obtained from honey in the manufacture of a medicament for the treatment of a skin condition on a subject in need thereof. Also disclosed herein is the use of an anti-inflammatory activity less than or equal to 10kDa fraction obtained from honey in the manufacture of a medicament for the treatment of a stomach ulcer and/or digestive condition in a subject in need thereof.

As noted above, the inventors have identified that a low molecular weight fraction from honey has strong anti-inflammatory effects and no immune-stimulatory effects. Being able to isolate compounds with anti-inflammatory activity allows the ability to produce medicaments for various uses including medical products, fortified foods, supplements and cosmetics. Anti-inflammatory activity may be useful in such medicaments to for example treat a sensitive topical wound and encourage healing whilst minimising pain. In an alternative illustrative example, the fraction may be formulated as a cream medicament applied to sunburnt skin as a means to calm the inflammation associated with the sunburn.

The anti-inflammatory effects noted above may be generalised. In very simple terms, the immune system may be characterised into three parts. One part consists of systems that detect pathogens, the second part signals this fact to other cells and the third part consists of the enzymes and antibodies that have a direct effect against the pathogen. Art that hints at a possible anti-inflammatory effect do not in the inventor's experience recognise or support a generalised effect. At best, art only supports a honey derived compound (not a fraction) having an inhibition effect on one enzyme in the third stage noted above. The art therefore at best only describes an inhibitory effect acting at the end of the inflammatory cascade and only highly specifically on one of the pathways. The inventors have observed anti-inflammatory effects in a more generalised form from the fraction noted. In one embodiment, the anti-inflammatory effects act on at least two effects (NOx and ROx) in neutrophils and monocytes. This indicates that the honey fraction effect or effects are due to one or several active components that affect the pathogen detection or signalling stage of the inflammatory response and therefore influence several of the pathways in the inflammatory reaction. This is quite different to the art that only supports or hints at a singular effect.

The skin condition in the above methods may be selected from: a burn, sunburn, bee sting, spider bite, eczema, psoriasis, localised inflammation skin or subcutaneous, a wound, and combinations thereof. Alternatively, the skin condition may be a cosmetic skin treatment.

In the above aspects, the subject may be human. Alternatively, the subject may be a non-human animal. As should be appreciated, humans and animals can equally be treated using the anti-inflammatory composition, as the physiology of an anti-inflammatory response may be similar between humans and at least mammals. Non-limiting examples of animals to which the composition may be administered includes horses, livestock including cattle, sheep and deer and companion animals such as cats and dogs.

The medicament may be formulated as: a cream, an ointment, a dressing, a spray, a gel, and an emulsion.

The honey from which the fraction is obtained may have a naturally higher concentration of phenolic compounds. In one embodiment, the honey may be of a floral origin substantially from the genus selected from: *Leptospermum, Kunzea, Trifolium, Knightea, Weinmannia, Metrosideros, Fagus, Myrtaceae* and combinations thereof. In selected embodiments, the honey from which the fraction is obtained may be substantially derived from *L. scoparium, K. ericoides,* or *Knightea excelsa,* or combinations thereof. The inventors have found that many honeys have at least some anti-inflammatory effects deriving from compounds in the <10kDa fraction, even honeys such as clover that are known to contain few phenolic based compounds. These findings are surprising since honeys like clover (Trifolium genus) and others tend to only be used for culinary purposes and not for medicament applications. Despite the above finding that all honeys have some anti-inflammatory activity deriving from the <10kDa fraction, some honeys have stronger anti-inflammatory effects than others most likely due to the different chemical profiles of these honeys based on floral origin and perhaps also geographical origin as well. Floral origin honeys that appear to have a slightly higher anti-inflammatory effects include manuka (*L. scoparium*), kanuka (*K. ericoides*) honeys and rewarewa honeys.

Further processing may occur to the <10kDa fraction isolated from honey. In one embodiment, any residual saccharides in the less than or equal to 10kDa fraction may also be removed. One example of a further processing step may be by processing the fraction via a solid phase extraction on C18 columns.

In one embodiment, the medicament itself may be a medical grade honey and the less than or equal to 10kDa fraction may be obtained from another honey and added to the medical grade honey. An example product exemplifying this embodiment may be a medical honey based wound dressing with an enhanced anti-inflammatory activity, the dressing being used in wounds where there is minimal if any microbial infection and the wound is in a healing phase. In a variation to the above, the medical grade honey used may be pre-selected based on that medical grade honey having an inherently higher natural concentration of less than or equal to 10kDa compounds and the addition of further compounds from an isolated fraction acts to enhance the already pre-existing compounds. The medical grade honey used may also be further pre-selected based on that honey having a lower concentration of immune stimulatory compounds. The immune stimulatory compounds may have a size greater than or equal to 30kDa. The immune stimulatory compounds may be selected from arabinogallactan proteins and/or apisimin protein.

In an alternative embodiment, the medical honey in the above embodiment may be substituted by use of a honey analogue solution and the fraction is added to the analogue to create an anti-inflammatory medicament.

The medicament described above may be formulated for topical administration. Example medicaments for topical administration may include wound dressings, putties, sheets, gels, creams, liquids, and ointments.

The medicament may be for treatment of wounds and be substantially incorporated into a dressing. As should be appreciated, wound dressings and aqueous based medicaments incorporating honey are well known and researched. Examples include those described in at least US7,714,183, US6,956,144, US11/106,473, US12/091,897 and US12/301,931. The anti-inflammatory effects of the fraction from honey described herein have considerable power to improve current wound dressings and medicaments (both those including honey and not including honey).

The dressing or aqueous based medicament may include at least one gelling agent. As noted above and in the art, gelling agents are advantageous for use with honey for wound applications. In particular, the gelling agents reduce the tackiness of the honey, yet provide a more cohesive structure such as a sheet structure or viscous gel that is easier to apply to a wound, skin region or mucosal lining. Gelling agents also have the advantage that they may be absorbent and work to move exudate away from a wound environment. This consequently avoids dilution of the honey fraction at the site.

The gelling agent may be selected from: an absorbent synthetic polymer, an absorbent natural based polymer, and combinations thereof.

The absorbent synthetic polymer may be selected from: any cross-linked sodium polyacrylate, polyacrylamide copolymer, ethylene maleic anhydride copolymer, carboxymethyl cellulose, polyvinyl alcohol copolymer, isobutylene-maleic anhydride copolymer, cross-linked polyethylene oxide, starch grafted copolymer or polyacrylonitrile, gauze, and combinations thereof.

The absorbent natural based polymer may be selected from: alginate, agar, natural based gums, and combinations thereof.

In the above embodiment where alginate is used, the alginate may be selected from: calcium alginate, sodium alginate, and combinations thereof.

The medicament may alternatively be formulated for oral administration. Example medicaments for oral administration include lozenges, elixirs, tablets, liquids, capsules, sprays, gels, ointments and fortified foods. Also disclosed herein is a method of producing an anti-inflammatory activity fraction substantially as described above from honey by the steps of:
(a) selecting a honey;
(b) diluting the honey in an aqueous solution and filtering the diluted honey via a less than or equal to 10kDa filter;
(c) collecting a less than or equal to 10kDa fraction wherein this fraction has anti-inflammatory activity.

The honey in step (a) may be selected based on an elevated natural concentration of less than or equal to 10kDa compounds compared to a standard baseline level for that floral origin of honey.

Selection in step (a) may be completed by techniques selected from: gas chromatography, HPLC, nitrogen oxide production from an in vitro assay, and/or the absence or reduction in pro-inflammatory cytokine production in an in vitro assay.

Filtration in step (b) may occur via: ultrafiltration, diafiltration, and combinations thereof. Also disclosed herein is a method of testing the anti-inflammatory potential of a honey by the steps of:
(a) stimulating a cell line with a suitable stimulant to induce an inflammatory response in the cells;
(b) preparing a <10kDa fraction from a honey by diluting the honey sample and collecting a <10kDa fraction by subjecting the diluted honey to a filtration separation step;
(c) applying the filtered honey containing the <10kDa fraction to the inflamed cells;
(d) measuring the reduction in cytokine production and/or nitrogen oxide production to determine the anti-inflammatory potential of the honey sample wherein the greater the reduction in inflammation as measured via cytokine production and/or nitric oxide production, the greater the anti-inflammatory activity of the honey tested.

The above method was developed by the inventors and provides a simple but effective way of screening honeys for the anti-inflammatory potential and then making decisions based on those findings regarding how the honey is subsequently used.

The stimulant used in step (a) may be the endotoxin lipopolysaccharide, LPS. LPS is known to elicit a strong and generalised immune response in animals and is used extensively in at least in-vitro cell cultures to stimulate cells and provide a control measure of stimulation. Filtration in step (b) may be via ultra-filtration.

The cytokines measured in step (d) may be TNF-α, IL-6, and combinations thereof. Advantages of the above anti-inflammatory medicaments, methods of isolation, methods of use and methods of analysis are varied. The source of the active compounds is a naturally occurring product able to be manufactured on a sustainable basis. The fraction is not anticipated to have side effects. The fraction may be formulated in a wide variety of ways for various methods of administration. Further the anti-inflammatory effects are significant and generalised suggesting good efficacy and a potentially broad range of applications/uses.

The embodiments described above may also be said broadly to consist in the parts, elements and features referred to or indicated in the specification of the application, individually or collectively, and any or all combinations of any two or more said parts, elements or features, and where specific integers are mentioned herein which have known equivalents in the art to which the embodiments relates, such known equivalents are deemed to be incorporated herein as of individually set forth,

### WORKING EXAMPLES

The above described fractions, methods and uses are now described by reference to specific examples.

### EXAMPLE 1

In this example, an experiment is described that was used to determine the presence of an anti-inflammatory effect from honey and what fraction is responsible for this effect.

### Methods and Materials Used

Firstly a cell line was established that would provide the in vitro test assay for stimulation and anti-inflammatory effects.

Four different floral origin honeys were selected for the experiment including, manuka, kanuka, clover and rewarewa honey were selected for the study. While no honey can be 100% from the stated origin, the honeys tested were known to be at least 80% of the floral origin indicated based on phenolic and other chemical profiling analysis.

A control honey analogue solution comprising a mixture of approximately 30-50% glucose, 30-50% fructose and approximately 18% water was also tested.

Samples of crude honey (unfractionated), as well as fractions of honey obtained by dilution and ultrafiltration were used to investigate the immuno-modulatory effect of honey.

Three size fractions were tested being molecular weight cut offs of greater than 30kDa (>30kDa) and less than 10kDa (<10kDa).

The type of cell that was cultured are the cells involved in the acute inflammatory response, white blood cells such as macrophages. In order to maintain reproducibility a continuous, immortalised cell line, called RAW 264.7 was used for the experiments Although inflammation is readily appreciated at a human level via for example redness, pain or fever, inflammation can be also characterised at a cellular level. Cellular inflammation may be characterised by production of various inflammatory mediators such as cytokines, chemokines or reactive nitrogen and oxygen species.

In this experiment, inflammation at a cellular level was studied in an in vitro system by culturing cells in artificial media and exposing them to microbes or microbial components followed by measuring the inflammatory mediators that are released into the medium.

At a cellular level, macrophages detect inflammatory stimuli through pattern recognition receptors, including toll like receptors. This is followed by intracellular signalling and leads to the production of inflammatory mediators such as pro-inflammatory cytokines (e.g. TNF-α, IL-6) and reactive nitrogen species (nitric oxide).

### Cytotoxicity

Before commencing use of honey in the trial, it was first necessary to find the highest usable concentration of honey and its fractions. Naturally, as honey concentration increases, cell death occurs. Hence, to see an effect from a part of honey, a dilution must be established where the cells are reactive but not completely overwhelmed.

To test various dilutions, RAW cells were incubated with honey or honey fractions with/of various concentrations for 24 hours followed by propidium iodide staining.

The viability of the cells was assessed as the ratio of positive stained (dead cells) over unstained (viable) cells and a viability of more than 95% was considered as non-toxic.

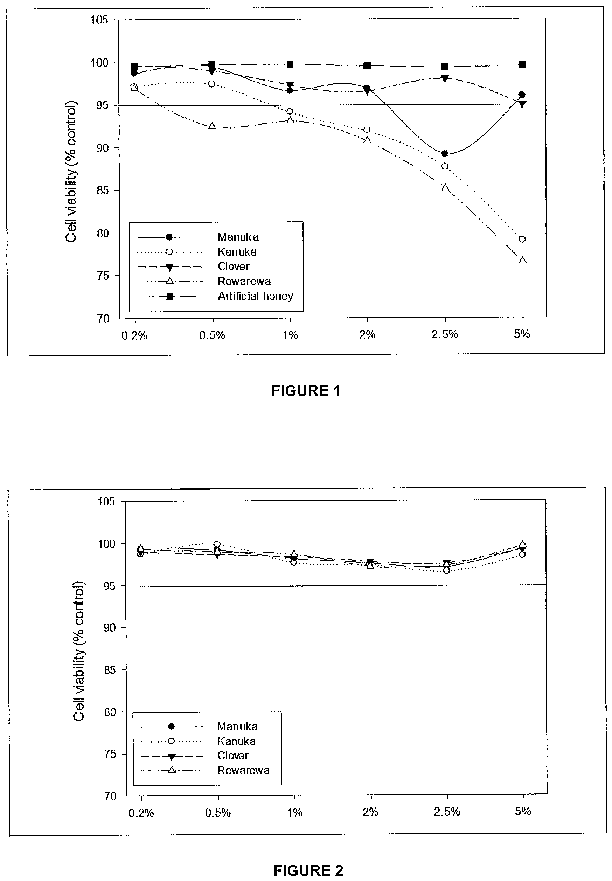

As anticipated and as shown in **Figure 1****,** crude honey has a cytotoxic effect when used at high concentrations. Interestingly this cytotoxic effect is independent of the honey sugar content, as artificial honey is non-cytotoxic at any concentration.

In contrast, honey fractions, obtained with ultrafiltration of the crude honey, are non-cytotoxic at any used concentration as shown in **Figure 2****.**

### Results - Crude Honey

RAW cells produce inflammatory mediators such as nitric oxide and tumour necrosis factor alpha (TNF-α) in response to stimulating agonists such as lipopolysaccharide (LPS).

In order to investigate the honey immune-modulating effects, RAW cells were treated with increasing concentrations of crude honey for 24 hours and the production of nitric oxide and TNF-α was measured in comparison to LPS induced production of nitric oxide and TNF-α.

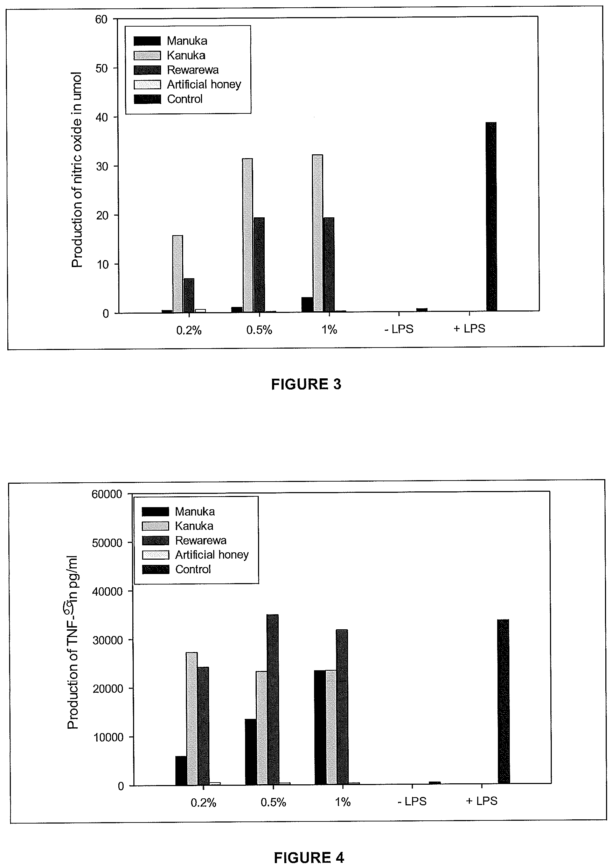

As shown in **Figure 3** and **Figure 4** crude honeys stimulate the immune system leading to production of nitric oxide and TNF-α in RAW cells. The stimulatory effect occurs for all of the honeys tested albeit with variation in stimulation levels however, artificial honey has no stimulating effect on the production of nitric oxide and TNF-α. Crude honey therefore has immune-stimulating or pro-inflammatory effects that are independent from its high sugar content - this effect supports earlier work by the applicant as published in earlier patents attributing such stimulatory effects to compounds such as arabinogalactan (AG) proteins.

### Results - Greater than 30kDa Fraction

The RAW cells were treated with a high (>30kDa) molecular weight fraction from the tested honeys in various concentrations for 24 hours and production of nitric oxide and TNF-α was measured and compared to a positive control LPS induced production of nitric oxide and TNF-α.

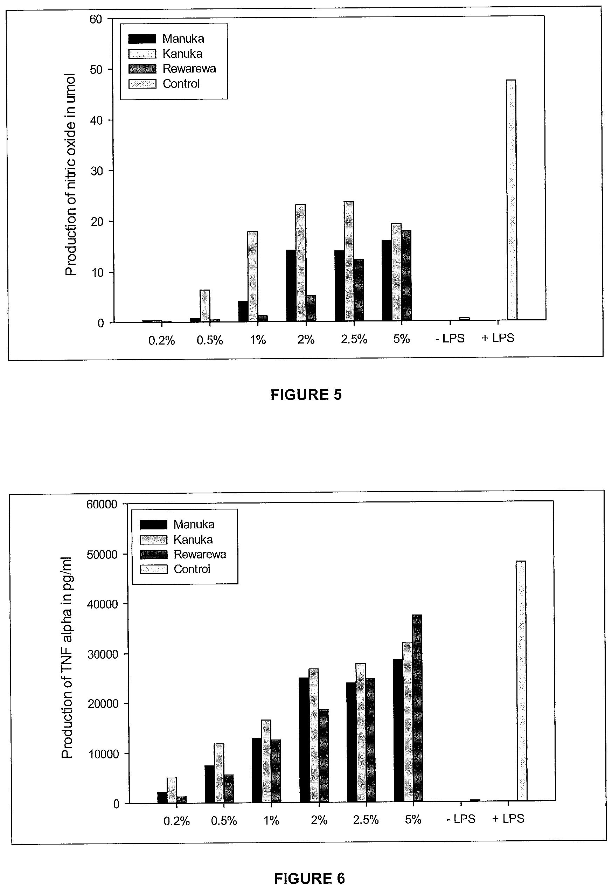

The high molecular weight fraction was anticipated to stimulate the production of nitric oxide and TNF-α given stimulating compounds would be in this fraction such as AG proteins. As shown in **Figure 5** and **Figure 6****,** stimulation did occur from this >30kDa fraction and the degree of stimulation were in a dose dependent manner

### Results - Less than 10kDa Fraction

The RAW cells were treated with a low (<10kDa) molecular weight fraction from the tested honeys in various concentrations for 24 hours and production of nitric oxide and TNF-α was measured and compared to a positive control LPS induced production of nitric oxide and TNF-α.

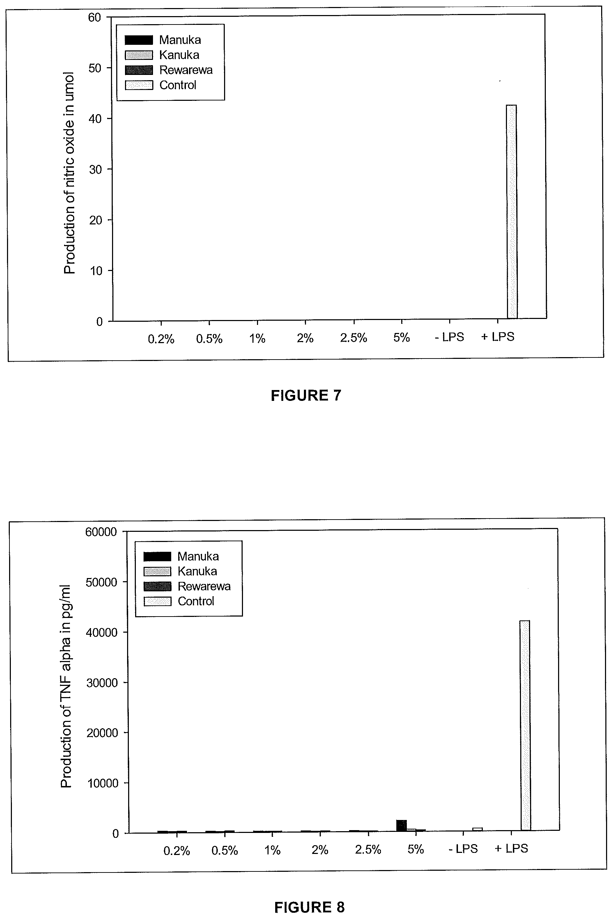

In this case, and as shown in **Figure 7** and **Figure 8****,** the <10kDa fraction has no stimulating effect on the production of nitric oxide and TNF-α showing that this fraction has no immune stimulation activity unlike the larger fraction.

### Results - Less than 10kDa Fraction Anti-Inflammatory Effects

In the above example, an absence of immune stimulation activity was observed for the <10kDa fraction. To confirm that the <10kDa fraction was not simply an inert fraction, a further trial was completed.

RAW cells were treated with the low molecular weight fraction of honey in various concentrations for 24 hours after stimulation with LPS. If the <10kDa fraction was anti-inflammatory, the inflammation markers observed for LPS should reduce. Nitric oxide and IL-6 were used as inflammatory markers and their levels measured in comparison with LPS induced production of nitric oxide and IL-6.

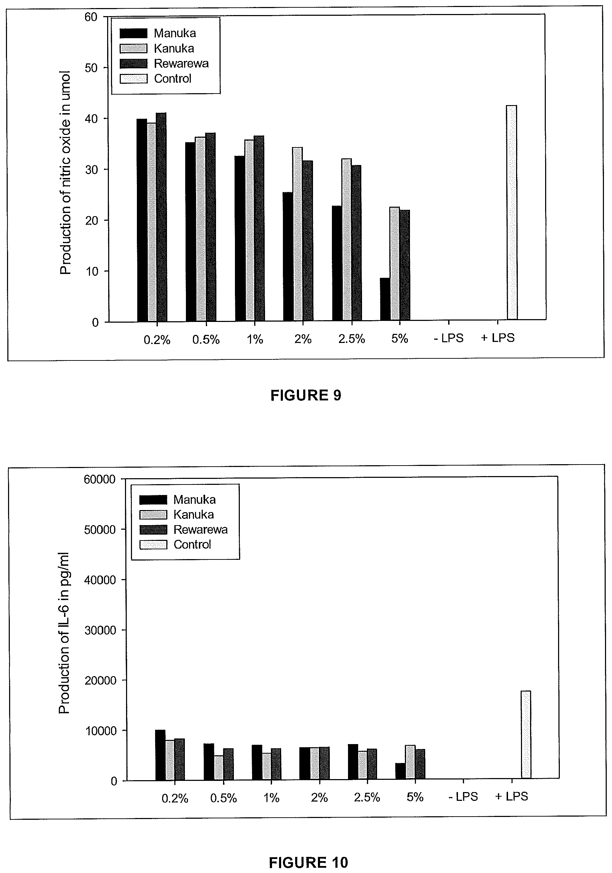

As shown in **Figure 9** and **Figure 10****,** the <10kDa fraction of honey reduces LPS induced production of nitric oxide and IL-6 in a dose dependent manner hence showing that the low molecular weight fraction has, not only a non-stimulating but also, an anti-inflammatory effect on the production of inflammatory mediators.

### EXAMPLE 2

As noted above, one method of measuring the potential anti-inflammatory activity of a honey is to measure the ability of the honey or fraction thereof in suppressing the production of nitric oxide from cells in an in-vitro cell culture. This example explains in detail, one method of completing this form of measurement.

### Initial steps comprise:

- Seeding 5x10⁵cells/200µl in a 96 well plate;
- Letting the cells grow for approximately 24 hours or until confluent;
- Treating the grown cells with a stimulant compound (dissolved in supplemented Dulbecco's Modified Eagel Medium (DMEM));
- Add honey or honey fractions in a concentration range from 0-5% (w/v) diluted in media;
- Subsequently sampling the cell supernatant and measuring the nitric oxide levels in the supernatant.

### Reagents:

- Sulfanilamide solution (1% sulphanilamide in 5% phosphoric acid);
- NED solution (N-1-naphylethylendiamine dihydrochloride in water);
- Nitrite standard (0.1M sodium nitrite).

### Prepare a nitrite standard reference curve as follows:

- Add 1ml of a 100µM nitrite solution into a DMEM medium;
- Dilute the standard (=0.1M sodium nitrite in milliQ water) 1:1000;
- Dispense 50µl of medium into the wells in rows B-H;
- Add 100µl of the 100µM nitrite solution into wells in row A;
- Perform 6 serial twofold dilutions (50µl/well) and discard 50µl from the 1.56µM set of wells (not nitrite in the last set of wells).

### Nitrite measurement (Griess reaction):

- Sulphanilamide solution and NED solution should be at room temperature (15-30mins);
- Add 50µl of each experimental sample to wells in duplicate or triplicate (supernatant medium from cells treated with honey, LPS, PMA etc.);
- Dispense 50µl of sulphanilamide solution to all experimental samples and wells for nitrite standard curve;
- Incubate 5-10mins at room temperature, protected from light;
- Add 50µl of NED solution to all wells;
- Incubate at room temperature for 5-10mins, protected from light (purple/,magenta) colour will begin to from immediately);
- Measure absorbance within 30mins, plate reader with filter between 520nm and 550nm.

### EXAMPLE 3

A protocol is provided below for measurement of the cytokines TNF-α and IL-6 via cytometric bead assays (CBA).
1) Reconstitute standards
- transfer lyophilized sphere (10000pg/ml) of assay protein into eppendorf tube;
- reconstitute with 100µl assay diluent (100000pg/ml);
- allow equilibration for at least 15mins at room temperature;
- gently mix by pipetting up and down;
- aliquot out 8µl in PCR tubes and freeze down at -20°C.

2) Prepare standards in eppendorf tubes
- thaw out aliquot with prepared standard (100000pg/ml);
- take 5µl of each reconstituted standard and top up to 100µl with Assay Diluent (standard is now 5000pg/ml) -> 5µl of TNF-α standard + 5µl of IL-6 standard + 90µl assay diluent;
- make serial dilution (50µl of mixed standards + 50µl of assay diluent);
- allow each dilution to equilibrate for 2mins until preparing the next dilution:

| Well label | Standard dilution | con. pg/ml |
|---|---|---|
| 1 | 0 | 0 |
| 2 | 1:512 | 10 |
| 3 | 1:256 | 20 |
| 4 | 1:128 | 40 |
| 5 | 1:64 | 80 |
| 6 | 1:32 | 156 |
| 7 | 1:16 | 312.5 |
| 8 | 1:8 | 625 |
| 9 | 1:4 | 1250 |
| 10 | 1:2 | 2500 |
| 11 | Top standard | 5000 |

3) Prepare protein capture beads:
- vortex capture bead stock vial for at least 15s;
- 25µl per well, 0.25µl for each capture bead;
- 48 samples, 11 standard dilutions, 5 extra;
- volume of capture bead diluent = (64x25µl)-(16µlx2) = 1568µl;
- 16µl of TNF-α beads + 16µl of IL-6 beads + 1568µl of diluent;
- pipette the capture beads and the diluent together for each cytokine (tube labelled mixed capture beads);
- store at 4°C, wrapped in tinfoil until use (leave 50µl for flow cytometry setup).

4) Dilute the samples in eppendorf tubes:
- dilute collected supernatants 1:10 with assay diluent;
- 5µl of sample with 45µl of assay diluent;
→ start assay:

- transfer 25µl of standard or sample into 96 well plate (leave at least one free row between each set of samples and the standard row to prevent spilling);
- vortex bead mix, add 25µl to each well;
- wrap plate in tinfoil and incubate for 1h on a shaker at 150rpm (room temperature):

| | | | | | | | | | | | | |
|---|---|---|---|---|---|---|---|---|---|---|---|---|
| A (standard) | 0 | 10 | 20 | 40 | 80 | 156 | 321.5 | 625 | 1250 | 2500 | 5000 | |
| B (samples) | most conc. | | | | | | | | | | | lowest conc. |
| C repeat | | | | | | | | | | | | |
| D (samples) | | | | | | | | | | | | |
| E repeat | | | | | | | | | | | | |
| F | | | | | | | | | | | | |
| G | | | | | | | | | | | | |

5) Prepare PE detection reagents:
- detection reagents have to be protected from light, wrap in tinfoil;
- volume of detection reagent diluent = (64x25µl)-(16µlx2) = 1568µl;
- 16µl of TNF-α detection reagent + 16µl of IL-6 detection reagent + 1568µl of diluent;
- pipette the detection reagents and detection reagent diluent into a tube (mixed PE detection reagents);
- wrap in tinfoil and incubate/store at 4°C until use.

6) after incubation 5):
- add 25µl of mixed PE detection reagent to each well:
- wrap plate in tinfoil and incubate for at least 1.5h on a shaker at 150rpm (room temperature).

7) Do instrument setup:
- after incubation, spin plate at 1300rpm for 5 mins;
- flick off supernatant;
- add 120µl of wash buffer;
- spin plate at 1300rpm for 5mins;
- discard supernatant;
- resuspend samples in 100µl of wash buffer;
- transfer into FACS tubes.

8) On the machine:
- vortex each sample before putting into the FACS;
- make sure the sample you want to measure is highlighted in the program;
- click load, as soon as it starts acquiring, click record data;
- after the run is finished click unload and carry on;
- in case of air bubbles, turn off the stream and turn in on again;
- in case of forgetting to switch to the next sample on the screen before running the next sample, click next sample as soon as it acquires data (it automatically stops), then start the run by clicking acquire and record data;
- always check if stopping gate: singlet and events to record: 900 (number depends on how many cytokines are measured).

### EXAMPLE 4

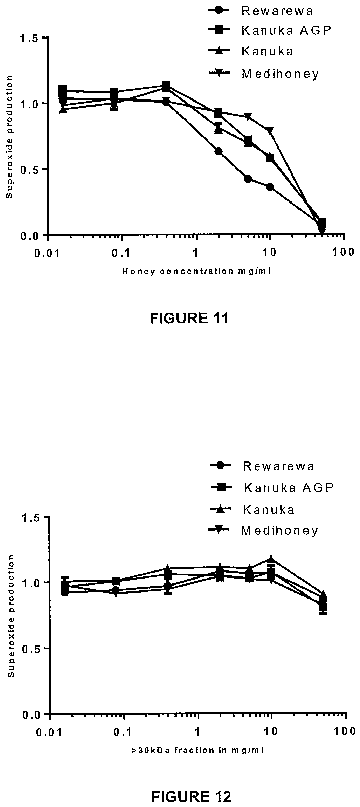

A further experiment was completed whereby human neutrophils were stimulated with PMA and exposed to honey or honey fractions and the oxidative burst reaction (production of superoxide) measured with the detection agent WST-1. As shown in **Figure 11****,** all honeys tested (rewarewa, kanuka and manuka-based Medihoney™ honey) showed an anti-inflammatory effect, by decreasing superoxide production with increased honey concentration.

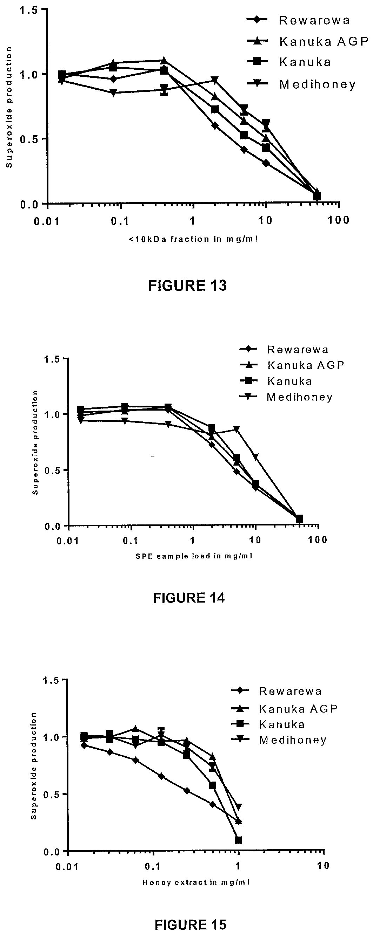

Honeys were then size fractioned by ultrafiltration and the larger than 30 kDa fraction and the smaller than 10 kDa fraction were tested. As shown in **Figure 12** and **Figure 13****,** the large fraction as anticipated in earlier work, showed no anti-inflammatory effect, while the small molecular weight fraction containes nearly all of the antiinflammatory effect observed for the whole honey.

Honey fractions were further purified with solid phase extraction on C18 columns. These columns bind phenolic components and molecules with a phenolic moiety. The extract should therefore be rich in honey phenolics. The samples were tested as described above. The highly purified honey phenolics show a strong inhibition of the superoxide production as illustrated in **Figure 14** and **Figure 15** which strongly suggests that phenolic compounds in the <10kDa fraction may be a main contributor to honey anti-inflammatory potential.

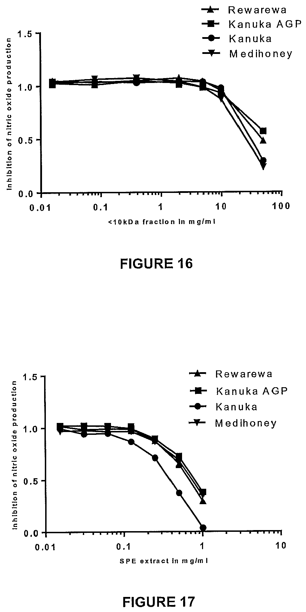

In a further test, RAW 264.4 cells were stimulated and the nitric oxide (NOx) production (another inflammatory reaction) was measured in the presence of varying honey fraction concentrations. As shown in **Figure 16** and **Figure 17****,** the smaller than 10 kDa fraction showed an inhibitory effect on the NOx production, while the purified extract from the above C18 column extraction showed an even stronger effect, suggesting that honey phenolics act on superoxide and nitric oxide production simultaneously.

## Claims

1. A medicament having anti-inflammatory activity and comprising as an active, a less than or equal to 10kDa molecular weight fraction obtained from honey using a less than or equal to 10kDa filter, the medicament being for use in the treatment of a skin condition of a subject in need thereof by administering the medicament to an affected skin site on a subject in need thereof, wherein the honey from which the fraction is obtained is substantially derived from manuka (*Leptospermum scoparium*), kanuka (*Kunzea ericoides*), or rewarewa (*Knightea excelsa*), or combinations thereof.

2. The medicament for use as claimed in claim 1 wherein the anti-inflammatory activity is a generalised anti-inflammatory effect.

3. The medicament for use as claimed in claim 1 or claim 2 wherein the skin condition is selected from: a burn, sunburn, bee sting, spider bite, eczema, psoriasis, localised inflammation skin or subcutaneous, a wound, and combinations thereof.

4. The medicament for use as claimed in any one of the above claims wherein the medicament is formulated as: a cream, an ointment, a dressing, a spray, a gel, and an emulsion.

5. The medicament for use as claimed in any one of the above claims wherein the medicament is formulated for topical administration as a: wound dressing; putty; sheet; gel; cream; liquid; or ointment.

6. A less than or equal to 10kDa molecular weight fraction obtained from honey using a less than or equal to 10kDa filter for use in the treatment of a skin condition on a subject in need thereof using anti-inflammatory activity, wherein the honey from which the fraction is obtained is substantially derived from manuka (*Leptospermum scoparium*), kanuka (*Kunzea ericoides*), or rewarewa (*Knightea excelsa*), or combinations thereof.

7. A medicament having anti-inflammatory activity and comprising as an active, a less than or equal to 10kDa molecular weight fraction obtained from honey using a less than or equal to 10kDa filter, the medicament being for use in the treatment of a stomach ulcer and/or digestive condition in a subject in need thereof, wherein the honey from which the fraction is obtained is substantially derived from manuka (*Leptospermum scoparium*)*,* kanuka (*Kunzea ericoides*), or rewarewa (*Knightea excelsa*), or combinations thereof.

8. The medicament for use as claimed in claim 7 wherein the anti-inflammatory activity is a generalised anti-inflammatory effect.

9. The medicament for use as claimed in claim 7 or claim 8 wherein the medicament is formulated for oral administration as a: lozenge; elixir; tablet; liquid; capsule; spray; gel; ointment; or fortified food.

10. A less than or equal to 10kDa molecular weight fraction obtained from honey using a less than or equal to 10kDa filter for use in the treatment of a stomach ulcer and/or digestive condition in a subject in need thereof using anti-inflammatory activity, wherein the honey from which the fraction is obtained is substantially derived from manuka (*Leptospermum scoparium*), kanuka (*Kunzea ericoides*), or rewarewa (*Knightea excelsa*), or combinations thereof.

11. A method of testing the anti-inflammatory potential of a honey by the steps of:
(a) stimulating a cell line with a suitable stimulant to induce an inflammatory response in the cells;
(b) preparing a <10kDa molecular weight fraction from a honey by diluting the honey sample and collecting a <10kDa molecular weight fraction by subjecting the diluted honey to a filtration separation step using a less than or equal to 10kDa filter, wherein the honey from which the fraction is obtained is substantially derived from manuka (*Leptospermum scoparium*), kanuka (*Kunzea ericoides*), or rewarewa (*Knightea excelsa*), or combinations thereof;
(c) applying the filtered honey containing the <10kDa molecular weight fraction to the inflamed cells;
(d) measuring the reduction in cytokine production and/or nitrogen oxide production to determine the anti-inflammatory potential of the honey sample wherein the greater the reduction in inflammation as measured via cytokine production and/or nitric oxide production, the greater the anti-inflammatory activity of the honey tested.

12. The method as claimed in claim 11 wherein the stimulant used in step (a) is lipopolysaccharide.

13. The method as claimed in claim 11 or claim 12 wherein filtration in step (b) is ultra-filtration.

14. The method as claimed in claim 11 or 12 wherein the filtration in step (b) is diafiltration.

15. The method as claimed in any one of claims 11 to 14 wherein the cytokines measured in step (d) are: TNF-α, IL-6, and combinations thereof.

## Patentansprüche

1. Medikament mit entzündungshemmender Aktivität, das als einen Wirkstoff einen von Honig unter Verwendung eines Filters von weniger als oder gleich 10kDa erhaltenen molekularen Gewichtsanteil von weniger als oder gleich 10kDa umfasst, wobei das Medikament zur Verwendung bei der Behandlung eines Hautleidens eines deren bedürfenden Empfängers durch Verabreichen des Medikaments an eine betroffene Hautstelle von einem dessen bedürfenden Empfänger dient, wobei der Honig, von dem der Anteil erhalten wird, im Wesentlichen von Manuka (*Leptospermum scoparium),* Kanuka (*Kunzea ericoides)* oder Rewarewa (*Knightea excelsa)* oder Kombinationen davon abgeleitet ist.

2. Medikament zur Verwendung nach Anspruch 1, wobei es sich bei der entzündungshemmenden Aktivität um eine verallgemeinerte entzündungshemmende Wirkung handelt.

3. Medikament zur Verwendung nach Anspruch 1 oder Anspruch 2, wobei das Hautleiden ausgewählt ist aus: einer Verbrennung, Sonnenbrand, Bienenstich, Spinnenbiss, Ekzem, Schuppenflechte, lokalisierter oder subkutaner Hautentzündung, einer Wunde und Kombinationen davon.

4. Medikament zur Verwendung nach einem der vorhergehenden Ansprüche, wobei das Medikament formuliert ist als: eine Creme, eine Salbe, ein Verband, ein Spray, ein Gel und eine Emulsion.

5. Medikament zur Verwendung nach einem der vorhergehenden Ansprüche, wobei das Medikament formuliert ist zur topischen Verabreichung als: ein Wundverband, eine Spachtelmasse; ein Tuch; ein Gel, eine Creme, eine Flüssigkeit oder eine Salbe.

6. Von Honig unter Verwendung eines Filters von weniger als oder gleich 10kDa erhaltener molekularer Gewichtsanteil von weniger als oder gleich 10kDa zur Verwendung bei der Behandlung eines Hautleidens eines deren bedürfenden Empfängers unter Verwendung einer entzündungshemmenden Aktivität, wobei der Honig, von dem der Anteil erhalten wird, im Wesentlichen von Manuka (*Leptospermum scoparium),* Kanuka (*Kunzea ericoides)* oder Rewarewa (*Knightea excelsa)* oder Kombinationen davon abgeleitet ist.

7. Medikament mit entzündungshemmender Aktivität, das als einen Wirkstoff einen von Honig unter Verwendung eines Filters von weniger als oder gleich 10kDa erhaltenen molekularen Gewichtsanteil von weniger als oder gleich 10kDa umfasst, wobei das Medikament zur Verwendung bei der Behandlung eines Magengeschwürs und/oder eines Verdauungszustands bei einem deren bedürfenden Empfänger dient, wobei der Honig, von dem der Anteil erhalten wird, im Wesentlichen von Manuka (*Leptospermum scoparium),* Kanuka (*Kunzea ericoides)* oder Rewarewa (*Knightea excelsa)* oder Kombinationen davon abgeleitet ist.

8. Medikament zur Verwendung nach Anspruch 7, wobei es sich bei der entzündungshemmenden Aktivität um eine verallgemeinerte entzündungshemmende Wirkung handelt.

9. Medikament zur Verwendung
nach Anspruch 7 oder Anspruch 8, wobei das Medikament
formuliert ist zur oralen Verabreichung als: eine Pastille; ein Elixier; eine Tablette; eine Flüssigkeit; eine Kapsel; ein Spray; ein Gel; eine Salbe; oder ein angereichertes Nahrungsmittel.

10. Von Honig unter Verwendung eines Filters von weniger als oder gleich 10kDa erhaltener molekularer Gewichtsanteil von weniger als oder gleich 10kDa zur Verwendung bei der Behandlung eines Magengeschwürs und/oder eines Verdauungszustands eines deren bedürfenden Empfängers unter Verwendung einer entzündungshemmenden Aktivität, wobei der Honig, von dem der Anteil erhalten wird, im Wesentlichen von Manuka (*Leptospermum scoparium),* Kanuka (*Kunzea ericoides)* oder Rewarewa (*Knightea excelsa)* oder Kombinationen davon abgeleitet ist.

11. Verfahren zum Testen des entzündungshemmenden Potenzials eines Honigs anhand der folgenden Schritte:
(a) Stimulieren einer Zelllinie mit einem geeigneten Stimulans, um eine Entzündungsreaktion in den Zellen hervorzurufen;
(b) Herstellen eines molekularen Gewichtsanteils <10kDa aus einem Honig durch Verdünnen der Honigprobe und Sammeln eines molekularen Gewichtsanteils <10kDa, indem der verdünnte Honig einem Filtrationstrennungsschritt unter Verwendung eines Filters von weniger als oder gleich 10kDa unterzogen wird, wobei der Honig, von dem der Anteil erhalten wird, im Wesentlichen von Manuka (*Leptospermum scoparium),* Kanuka (*Kunzea ericoides)* oder Rewarewa (*Knightea excelsa)* oder Kombinationen davon abgeleitet ist;
(c) Anwenden des gefilterten Honigs, der den molekularen Gewichtsanteil <10kDa enthält, auf die entzündeten Zellen;
(d) Messen der Verringerung bei der Cytokinproduktion und/oder Stickstoffoxidproduktion, um das entzündungshemmende Potenzial der Honigprobe zu bestimmen, wobei je größer die Verringerung der Entzündung, wie anhand der Cytokinproduktion und/oder Stickstoffoxidproduktion gemessen, desto größer die entzündungshemmende Aktivität des getesteten Honigs.

12. Verfahren nach Anspruch 11, wobei es sich bei dem in Schritt (a) verwendeten Stimulans um Lipopolysaccharid handelt.

13. Verfahren nach Anspruch 11 oder Anspruch 12, wobei es sich bei der Filtration in Schritt (b) um Ultrafiltration handelt.

14. Verfahren nach Anspruch 11 oder 12, wobei es sich bei der Filtration in Schritt (b) um Diafiltration handelt.

15. Verfahren nach einem der Ansprüche 11 bis 14, wobei es sich bei den in Schritt (d) gemessenen Cytokinen um TNF-α, IL-6 und Kombinationen davon handelt.

## Revendications

1. Médicament ayant une activité anti-inflammatoire et comprenant, comme ingrédient actif, une fraction de poids moléculaire inférieur ou égal à 10 kDa obtenue de miel avec utilisation d'un filtre inférieur ou égal à 10 kDa, le médicament devant être utilisé dans le traitement d'une affection cutanée d'un sujet qui en a besoin par administration du médicament à un site cutané affecté sur un sujet qui en a besoin, le miel dont la fraction est obtenue étant essentiellement dérivé de manuka (*Leptospermum scoparium*), kanuka (*Kunzea ericoides*), ou rewarewa (*Knightea excelsa*), ou de combinaisons de ceux-ci.

2. Médicament pour utilisation selon la revendication 1, dans lequel l'activité anti-inflammatoire est un effet anti-inflammatoire généralisé.

3. Médicament pour utilisation selon la revendication 1 ou la revendication 2, dans lequel l'affection cutanée est sélectionnée entre : brûlure, coup de soleil, piqûre d'abeille, morsure d'araignée, eczéma, psoriasis, inflammation localisée cutanée ou sous-cutanée, plaie et des combinaisons de ceux-ci.

4. Médicament pour utilisation selon l'une quelconque des revendications précédentes, le médicament étant formulé sous forme de: crème, onguent, pansement, pulvérisation, gel et émulsion.

5. Médicament pour utilisation selon l'une quelconque des revendications précédentes, le médicament étant formulé pour administration topique sous forme de : pansement pour plaie ; pâte ; feuille ; gel ; crème ; liquide ; ou onguent.

6. Fraction de poids moléculaire inférieur ou égal à 10 kDa obtenue de miel avec utilisation d'un filtre inférieur ou égal à 10 kDa pour utilisation dans le traitement d'une affection cutanée sur un sujet qui en a besoin, en utilisant une activité anti-inflammatoire, où le miel dont la fraction est obtenue est essentiellement dérivé de manuka (*Leptospermum scoparium*), kanuka (*Kunzea ericoides*), ou rewarewa (*Knightea excelsa*), ou de combinaisons de ceux-ci.

7. Médicament ayant une activité anti-inflammatoire et comprenant, comme ingrédient actif, une fraction de poids moléculaire inférieur ou égal à 10 kDa obtenue de miel avec utilisation d'un filtre inférieur ou égal à 10 kDa, le médicament devant être utilisé dans le traitement d'un ulcère à l'estomac et/ou d'une affection digestive chez un sujet qui en a besoin, le miel dont la fraction est obtenue étant essentiellement dérivé de manuka (*Leptospermum scoparium*), kanuka (*Kunzea ericoides*), ou rewarewa (*Knightea excelsa*), ou de combinaisons de ceux-ci.

8. Médicament pour utilisation selon la revendication 7, dans lequel l'activité anti-inflammatoire est un effet anti-inflammatoire généralisé.

9. Médicament pour utilisation selon la revendication 7 ou la revendication 8, le médicament étant formulé pour administration orale sous forme de : pastille ; élixir ; comprimé ; liquide ; capsule ; pulvérisation ; gel ; onguent ; ou aliment enrichi.

10. Fraction de poids moléculaire inférieur ou égal à 10kDa obtenue de miel avec utilisation d'un filtre inférieur ou égal à 10 kDa pour utilisation dans le traitement d'un ulcère à l'estomac et/ou d'une affection digestive chez un sujet qui en a besoin en utilisant une activité anti-inflammatoire, le miel dont la fraction est obtenue étant essentiellement dérivé de manuka (*Leptospermum scoparium*), kanuka (*Kunzea ericoides*), ou rewarewa (*Knightea excelsa*), ou de combinaisons de ceux-ci.

11. Procédé pour tester le potentiel anti-inflammatoire d'un miel par les étapes consistant à :
(a) stimuler une lignée cellulaire avec un stimulant approprié pour susciter une réaction inflammatoire dans les cellules ;
(b) préparer une fraction de poids moléculaire <10 kDa à partir d'un miel en diluant l'échantillon de miel et en prélevant une fraction de poids moléculaire <10 kDa en soumettant le miel dilué à une séparation par filtration au moyen d'un filtre inférieur ou égal à 10 kDa, le miel dont la fraction est obtenue étant essentiellement dérivé de manuka (*Leptospermum scoparium*), kanuka (*Kunzea ericoides*), ou rewarewa (*Knightea excelsa*), ou de combinaisons de ceux-ci ;
(c) appliquer le miel filtré contenant la fraction de poids moléculaire <10 kDa aux cellules enflammées ;
(d) mesurer la réduction de production de cytokine et/ou la production d'oxyde d'azote pour déterminer le potentiel anti-inflammatoire de l'échantillon de miel, l'activité anti-inflammatoire du miel testé étant d'autant plus importante que la réduction de l'inflammation mesurée par la production de cytokine et/ou la production d'oxyde nitrique est importante.

12. Procédé selon la revendication 11, dans lequel le stimulant utilisé dans l'étape (a) est un lipopolysaccharide.

13. Procédé selon la revendication 11 ou la revendication 12, dans lequel la filtration de l'étape (b) est l'ultra-filtration.

14. Procédé selon la revendication 11 ou 12, dans lequel la filtration de l'étape (b) est la diafiltration.

15. Procédé selon l'une quelconque des revendications 11 à 14, dans lequel les cytokines mesurées à l'étape (d) sont : TNF-α, IL-6, et des combinaisons de celles-ci.
